# EUROPÄISCHE PATENTSCHRIFT

(11) **EP 1 413 321 B1**
(45) Veröffentlichungstag und Bekanntmachung des Hinweises auf die Patenterteilung: **04.01.2006**
(21) Anmeldenummer: 03090348.8
(22) Anmeldetag: 16.10.2003
(51) Int. Cl.: A61L 27/12

(54) **Phosphathaltiger Knochenersatzwerkstoff mit kristallinen und röntgenamorphen Phasen**
Phosphate containing bone substitute product with crystalline and amorphous phases
Matériau pour prothèse osseuse à base de phosphate comprenant des phases cristallines et amorphes

(30) Priorität: 21.10.2002 DE 10249627
(43) Veröffentlichungstag der Anmeldung: 28.04.2004
(73) Patentinhaber: BAM Bundesanstalt für Materialforschung und -prüfung, 12205 Berlin (DE)
(72) Erfinder: Berger, Georg, 16341 Zepernick (DE); Spitzer, Andrea, 10827 Berlin (DE); Jäger, Christian Prof., 07749 Jena (DE); Pauli, Jutta, 12555 Berlin (DE); Gildenhaar, Renate, 13086 Berlin (DE)
(74) Vertreter: Walter, Wolf-Jürgen

(56) Entgegenhaltungen:
- WO-A-91/07357
- DE-C- 19 744 809
- US-A- 3 922 155
- KNABE C ET AL: "Morphological evaluation of osteoblasts cultured on different calcium phosphate ceramics" BIOMATERIALS, ELSEVIER SCIENCE PUBLISHERS BV., BARKING, GB, Bd. 18, Nr. 20, 1997, Seiten 1339-1347, XP004091898 ISSN: 0142-9612

## Beschreibung

Die Erfindung betrifft ein röntgenamorph-kristallines Material mit hoher Löslichkeit, das sowohl als bioaktiver Knochenersatzwerkstoff, so z.B. als Beschichtung von metallischen Prothesenstielen durch thermisches Spritzen, durch rf-Sputtering, als auch als Substratmaterial in der Biotechnologie, speziell dem Tissue Engineering, z.B. auch als keramische Folie, als kompakter oder poröser, d.h. spongiosa-ähnlicher, "scaffold"artiger, Formkörper Anwendung finden kann. Die Erfindung betrifft auch ein Herstellungsverfahren.

Anorganische Materialien mit hoher Resorbierbarkeit sind an sich bekannt. Auch Werkstoffe, die ihren speziellen Einsatz als bioaktive Knochenersatzwerkstoffe finden und eine schnelle Löslichkeit besitzen, sind in der Literatur beschrieben. Beispielsweise wurde ständig über den erfolgreichen klinischen Einsatz von Keramiken mit den Hauptkristallphasen alpha- oder beta-Tricalciumphosphat (TCP) berichtet Zudem gab es auch vergleichende Untersuchungen dieser beiden TCP-Phasen im Tierversuch. Aus EP 237043 ist bekannt, dass an der Oberfläche von aus alpha-TCP hergestellten Granulaten Dicalciumphosphat enthalten ist, was besonders in der Anfangsphase nach der Implantation eine höhere Löslichkeit aufwies als das reine Kernmaterial aus alpha-TCP.

Übertroffen wurde deren chemische Löslichkeit durch ebenfalls bioaktive Werkstoffe auf der Basis von Caiciumphosphaten, die zusätzlich Oxide des Kaliums, Natriums, Magnesiums und/oder Siliciums enthalten (EP 541546 B1) und deren glasigkristallines Material sich auf die folgende Hauptkristallphasen gründet Phase X, Rhenanit, Phase nach Ando (Phase A) bzw. von diesen zuvor genannten Phasen abgeleitete Mischkristalle enthält.

Die WO 91/07357 offenbart Oxidzusammensetzungen, die als Knochenersatzmaterial mit schneller Löslichkeit einsetzbar sind, bestehend aus einer kristallinen X-Phase und je nach Schmelzbereich anderen Phasen, enthaltend CaO, Na₂O, K₂O, MgO, P₂O₅, SiO₂ und gegebenenfalls Sulfate. Die Zusammensetzung wird gesintert oder geschmolzen und danach je nach gewünschter Kristallphase entsprechend abgekühlt, wobei Löslichkeiten im Bereich von 3-15 mg/g erhalten werden. Die DE 19744809 C offenbart einen glasig-kristallinen Glaskörper mit Ca-K-Na-Phosphat-Hauptkristallphasen mit schneller Löslichkeit als Knochenersatzwerkstoff hergestellt durch Zusammenschmelzen zweier Gläser, wobei eines der Gläser 20-55% CaO, 5-25 Na₂O, 0,01-20 K₂O, 0-15 MgO, 30-50 P₂O₅ und 0-15 SiO₂ enthält. Die Porosität wird durch Auslaugung der Borosilicatglasphase auf einen Rest von 0,05-2 Gew% erhalten. US-A-3922155 offenbart einen Prozeß zur Herstellung von biokompatiblen Glaskeramiken, die das Schmelzen einer SiO₂, Na₂O, K₂O, MgO, CaO und Ca₃(PO4)₂-Mischung sowie weitere Keramisierungsschritte umfaßt. Dabei werden für Knochen- und Zahnersatz günstige Apatitstrukturen ausgebildet. In BIOMATERIALS Bd. 18, Nr. 20 (1997)1339-1347 wird der Einsatz von Ca₂KNa(PO₄)₂ Als Knochenersatzwerkstoff beschrieben, jedoch ohne Informationen über Herstellung und Phasen.

Die Erfindung hat die Aufgabe, ein röntgenamorph-kristallines Material bereitzustellen, das einen überwiegend direkten, bindegewebsfreien Knochenverbund bzw. die Ex-vivo-Kultivierung von Knochenzellen ermöglicht und das sich im Kontakt mit Knochengewebe auflöst und dabei verbessert einstellbare hohe Löslichkeiten und bei Kompositen zugleich an bestimmte Stähle angepasste Ausdehnungskoeffizienten aufweist. Eine weitere Aufgabe besteht darin, ein Herstellungsverfahren für das Material sowie Hilfsmittel für die Herstellung zu entwickeln.

Erfindungsgemäß besteht der Knochenersatzwerkstoff aus kristallinen und röntgenamorphen Phasen und enthält
nach ³¹P-NMR-Messungen Q₀-Gruppen von Orthophosphat und Q₁-Gruppen von Diphosphat, wobei die Orthophosphate respektive Q₀-Gruppen, bezogen auf den Gesamtphosphorgehalt des fertigen Werkstoffes, 70 bis 99,9 Gew-% betragen, und die Diphosphate respektive Q₁-Gruppen, bezogen auf den Gesamtphosphorgehalt des fertigen Werkstoffes, 0,1 bis 30 Gew-% betragen, und
wobei nach röntgendiffraktometrischen Messungen, bezogen auf das Gesamtgewicht des fertigen Werkstoffes, 30 bis 99,9 Gew-% einer Hauptkristallphase aus Ca₂K₁₋ₓNa₁₊ₓ(PO₄)₂ , worin x=0,1 bis 0,9 ist, enthalten sind, und als Nebenkristallphase, bezogen auf das Gesamtgewicht des fertigen Werkstoffes, 0,1 bis 20 Gew-% eines Stoffes enthalten sind, ausgewählt aus der Gruppe, bestehend aus Na₂CaP₂O₇, K₂CaP₂O₇, Ca₂P₂O₇ und Gemische davon, und
wobei die röntgenamorphen Phasen neben der Hauptkristallphase insgesamt 0,1 bis 70 Gew-% betragen, bezogen auf das Gesamtgewicht des fertigen Werkstoffes, erhältlich durch
Vermischen von Rohstoffen, enthaltend (in Gew-%) 25-50 CaO, 1-20 Na₂O, 0,5-20 K₂O, 0-13 MgO, 0-10 SiO₂ und Behandlung mit H₃PO₄ entsprechend einem Anteil von 30-55 P₂O₅, wobei SiO₂ oder MgO oder ein Gemisch davon wenigstens 1 Gew-% beträgt, das Gemenge homogenisiert und getrocknet wird und einer stufenweisen Temperaturbehandlung von jeweils 1-2h bei 350-450 °C, 750-850 °C und 950-1050 °C unterworfen wird, und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

In der Nebenkristallphase sind zwar die Diphosphate bevorzugt, jedoch können auch ein oder mehrere der Stoffe NaPO₃, KPO₃und Gemische davon enthalten sein, wobei die Kettenphosphate NaPO₃ und KPO₃ nach ³¹P-NMR-Messungen als Q₂-Gruppen nachzuweisen sind. Der Anteil der Kettenphosphate beträgt 0,1 bis 10 Gew-%, vorzugsweise 0,1 bis 4 Gew-% .

Weiterhin kann in der Nebenphase entsprechend dem SiO₂-Anteil eine Silicatphase in einem Anteil bis 6 Gew-% enthalten sein.

In der o.g. Hauptkristallphase sowie in den Bestandteilen der Nebenkristallphase kann Magnesium enthalten sein bis zu einem Anteil von 10 Gew-%, berechnet als MgO und bezogen auf das Gewicht des fertigen Werkstoffs.

Der Anteil der Orthophosphatphase als Q₀-Gruppen kann bevorzugt im Bereich von 75 bis 99 Gew-% liegen, insbesondere im Bereich von 78 bis 95 Gew-%.

Der Anteil der Diphosphatphase als Q₁-Gruppen kann bevorzugt im Bereich von 1 bis 22 Gew-% liegen, insbesondere im Bereich von 5 bis 16 Gew-%.

Die Zusammensetzung des röntgenamorph-kristallinen Materials mit hoher Löslichkeit auf Basis von CaO, P₂O₅, Na₂O, K₂O, MgO und ggf. auch SiO₂ liegt im Bereich von (in Gew-%):

| | | | |
|---|---|---|---|
| 30 | bis | 55 | P₂O_{5;} 25 bis 50 CaO; |
| 1 | bis | 20 | Na₂O; 0,5 bis 20 K₂O; |
| 0 | bis | 13 | MgO; 0 bis 10 SiO₂ ; |

wobei MgO oder SiO₂ oder ein Gemisch davon wenigstens 1 Gew-% betragen.

Ein bevorzugtes röntgenamorph-kristallines Material enthält folgende Bestandteile: in Gew-%: 35 bis 48 P₂O₅, 28 bis 38 CaO, 2,5bis 15 Na₂O, 1,5 bis 18 K₂O, 0,1 bis 4 MgO, 0,0 bis 3 SiO₂. Eine spezielle bevorzugte Ausführungsform enthält 40 bis 52 P₂O₅, 28 bis 33 CaO, 8,5 bis 13 Na₂O, 9,5 bis 15 K₂O,1,5 bis 3 MgO, 0,1 bis 4 SiO₂.

Der hier verwendete Begriff "röntgenamorph-kristallines" Material ist im allgemeinen nicht eindeutig definierbar. Unter röntgenamorph wird hier ein Material verstanden, dessen Struktur mit der üblichen XRD (Röntgendiffraktometrie) nicht mehr erfasst werden kann. Dabei kann es sich um sehr kleine geordnete Bereiche (mikrokristallin) als auch statistisch ungeordnete Bereiche handeln. Im Gegensatz zu XRD kann durch die ³¹P-NMR-Ergebnisse die Existenz jeder kristallinen Phase erfasst werden. Deshalb kann es bei der Mengenabschätzung zwischen NMR- und XRD-Ergebnissen zu gravierenden Unterschieden kommen. Besonders die Diphosphat- und Kettenphosphatanteile scheinen im vorliegenden Fall für dieses Phänomen symptomatisch zu sein; es werden in der Regel mit den ³¹P-NMR-Messungen deutlich höhere Anteile bestimmt als mit XRD. Mit XRD werden teilweise sogar keine Anteile bestimmt. Dies zeigt eindrucksvoll, warum die ³¹P-NMR-Messungen für die Charakterisierung und letztlich Herstellung der erfindungsgemäßen Werkstoffe eine wesentliche Voraussetzung bilden. Die XRD-Messungen erfolgten mit einem PW 1710, Philipps, NL (CuK radiation).

Es können daher sowohl kristalline als auch röntgenamorphe Phasen innig vermischt vorliegen. Für die vorliegende Erfindung ist es ohne Belang, ob eine Phase neben der anderen vorliegt, oder ob eine Phase die andere umhüllt. Als "Hauptkristallphase" wird hier eine über Röntgendiffraktion ermittelte kristalline Phase bezeichnet, deren Mengenanteil wenigstens doppelt so groß ist, wie der einer Nebenphase, wobei Konzentrationen von 20% und darunter, vorzugsweise unter 15 Gew-%, als Nebenphasen bezeichnet werden.

Zum besseren Verständnis muss noch darauf hingewiesen werden, dass zwar als Hauptkristallphase "Ca₂KNa(PO₄)₂"identifiziert werden kann. Es gibt jedoch in den einzelnen Zusammensetzungen teilweise erhebliche Linienverschiebungen , die auf das wechselnde Verhältnis von Natrium zu Kalium bzw. auf den Einbau anderer lonen (wie Mg²⁺ oder SiO₄⁴⁻) zurückzuführen sind, so dass die Formel "Ca₂K₁₋ₓNa₁₊ₓ(PO₄)₂, wobei x=0,1-0,9 ist" einzusetzen ist. Höhere Anteile Na sind bevorzugt, z.B. x=0,2-0,9.

Überraschenderweise wurde nun gefunden, dass eine besonders hohe Löslichkeit dann vorliegt, wenn neben den Hauptkristallphasen sowie röntgenamorphen Anteilen von Orthophosphaten insbesondere kristalline Diphosphate wie Na₂CaP₂O₇, K₂CaP₂O₇ und/oder Ca₂P₂O₇ oder überwiegend sogar auch röntgenamorphe Anteile an Diphosphaten im Schmelzprodukt vorliegen. Ferner wurde überraschenderweise gefunden, dass sich diese Aussage durch ³¹P-NMR-Messungen eindeutig quantifizieren lasst.

Die ³¹P-NMR-Messungen, die mit einem supraleitenden Fourier NMR Spektrometer Avance DMX400 WB der Fa. Bruker BioSpin GmbH (Deutschland) durchgeführt wurden, zeigten, dass das Material aus 70 bis 99,9 Gew-% Orthophosphat besteht, gebildet aus Calcium sowie ggf. Natrium, Kalium und Magnesium, wobei dieser Orthophosphat-Anteil durch ³¹P-NMR-Messungen über Q₀-Gruppen nachgewiesen wird und sich auf kristallines und/oder röntgenamorphes Material in seiner Gesamtheit bezieht. Ferner wurden 0,1 bis 30 Gew-% Diphosphat gefunden, gebildet aus Calcium sowie ggf. Natrium, Kalium und Magnesium, wobei dieser DiphosphatAnteil nachweislich durch ³¹P-NMR-Messungen (Q₁-Gruppen) festzustellen ist und sich auf kristallines und/oder röntgenamorphes Material in seiner Gesamtheit bezieht.

Weiterhin vorteilhaft enthalten sein können 0,1 bis 10 Gew-% Kettenphosphat, bestehend aus Natrium und/oder Kaliumphosphat, wobei dieser Kettenphosphat-Anteil als Q₂-Gruppen nachweislich durch ³¹P-NMR-Messungen nachgewiesen wird und sich insbesondere auf amorphes und/oder kristallines Material in seiner Gesamtheit bezieht. Ferner kann in Abhängigkeit vom gewählten SiO₂-Zusatz 0,1 bis 10 Gew-% einer Silicatphase enthalten sein. Weiterhin kann Ca₅Na₂(PO₄)₄ enthalten sein, was jedoch nicht bevorzugt ist.

Es wurde ferner überraschenderweise gefunden, dass die Anwesenheit von Di- bzw. Kettenphosphaten, vorzugsweise jedoch Diphosphaten, den gewünschten Effekt in Bezug auf eine deutliche Verbesserung der Löslichkeit bewirkt, wie das Beispiel 3 im weiteren belegen wird.

Die Diphosphatanteile resultieren aus einem vergleichsweise hohen Phosphatanteil im Vergleich zu den übrigen Bestandteilen. Dieser könnte auch ursächlich dafür verantwortlich sein, dass die erfindungsgemäßen Zusammensetzungen im Vergleich zu bekannten resorbierbaren Werkstoffen sehr leicht einschmelzen und eine dünnflüssige Schmelze ergeben. Eine solche dünnflüssige Schmelze hat den Vorteil, dass sie besser zu verarbeiten ist. Dies trifft z.B. auf eine Fritte der Schmelze oder auf ein direktes Verblasen der Schmelze usw. zu.

Ferner wurde überraschenderweise gefunden, dass durch die Anwesenheit von Diphosphaten nach Lagerung in deionisiertem Wasser der Werkstoff (das röntgenamorph-kristalline Material) nach anfänglich stark alkalischer Reaktion seinen lonenaustrag in Richtung physiologische pH-Werte (7,4) stärker verändert im Vergleich zu Werkstoffen, die kein Diphosphat enthalten. Dieser pH-Wert-Shift macht den Werkstoff auch für die Biotechnologie respektive Tissue Engineering interessant.

Dieses Merkmal lässt sich dadurch verstärken, dass ein Formkörper (kompakt oder offenporig) durch Kochen in deinonisiertem Wasser (37 - 90°C) oberflächlich ausgelaugt wird, so dass der auf diese Weise behandelte Werkstoff bzw. Formkörper nach dieser Behandlung deutlich geringere pH-Werte aufweist. Als Ursache dafür könnte eine Verarmung der alkalischen Kationen im oberflächennahen Bereich in Betracht gezogen werden. Dieser Vorgang kann durch Kochen in einem Reaktor, vorteilhaft unter einem Druck bis zu 10 bar, beschleunigt werden. Eine solche Ausführungsform der Erfindung ist bevorzugt.

Ein vorteilhaftes Merkmal des erfindungsgemäßen Materials ist es, dass in Abhängigkeit von der gewählten Zusammensetzung die Löslichkeit in relativ weiten Bereichen eingestellt werden kann und zwar von 30 bis 500 µg/mg Gesamtlöslichkeit bezogen auf das Ausgangsmaterial, wenn die Prüfung in 0,2M TRIS-HCI-Puffer-Lösung bei pH=7,4, T=37°C, an einer Komfraktion von 315 - 400µm, 120h lang bei einem Verhältnis von 50mg Probeneinwaage zu 40ml Pufferlösung erfolgte.

Der erfindungsgemäße Werkstoff wird hergestellt, indem die für die Gemengebildung geeignete Substanzen im Konzentrationsbereich (gezogen auf das Gesamtgewicht des Werkstoffs) 30 - 55 Gew-% CaO, 35 - 50 Gew-% P₂O₅, 1 - 20 Gew-% Na₂O, 0,5 - 20 Gew-% K₂O und 0,1 - 5 Gew-% MgO sowie gegebenenfalls bis zu 5 Gew-% SiO₂ kombiniert werden, und in mehrstufigen Temperaturbehandlungsprogrammen mit Haltestufen im Bereich von 200 bis 1500 °C, und zwar 1-2 h bei 350 - 400°C, 750 - 850°C und 950 - 1050°C, z.B. jeweils 1h bei 400, 800 und 1000 °C in einem geeigneten Tiegelmaterial, z. B. bestehend aus einer Pt/Rh-Legierung, bei 1550 bis 1650 °C zum Schmelzen gebracht werden. Die Schmelze wird , vorteilhaft nach einer Haltezeit von 10 bis 60 min, vergossen, und die erstarrte Schmelze wird je nach Verwendungszweck an der Luft (spontane Abkühlung) oder im Kühlofen mit temperaturprogrammierter Abkühlung von z.B. 1 bis 20 Grad/min auf Raumtemperatur abgekühlt. Sie kann auch verblasen werden, wodurch kugelförmige Granulate direkt aus dem Schmelzfluss entstehen. Während des Abkühlens der Schmelzen erfolgt stets eine spontane Kristallisation. Als Gemengebestandteile können Oxide, Carbonate, Hydrogenphosphate und/oder Ortho-Phosphorsäure verwendet werden. Die ³¹P-NMR-Messungen zeigen dabei Unterschiede in den Spektren auf, die auf die verwendeten Rohstoffe Rückschlüsse zulassen bzw. respektive deren geringfügige Beimengungen an Eisen- bzw. Manganoxiden anzeigen. Bevorzugte Schmelztemperaturen liegen bei 1590 - 1650°C.

Nach der Abkühlung wird der Werkstoff granuliert und als knochenersatz werkstoff eingesetzt, er kann jedoch beispielsweise auch aufgemahlen, mit üblichen Sinterhilfsmitteln versetzt und sodann zu Formkörpern isostatisch verpresst werden, um nach dem Sintem einen möglichst dicht gebrannten keramischen Scherben zu ergeben. Die Sintertemperaturen liegen im allgemeinen bei 900 - 1200°C.

Der erfindungsgemäß hergestellte Werkstoff kann beispielsweise auch aufgemahlen, mit üblichen Sinterhilfsmitteln versetzt und zu Schlicker verarbeitet werden, dieser auf einen Polyurethanschwamm aufgebracht und in mehreren Sinterstufen so hoch gesintert werden, dass der Polyurethanschwamm und die Sinterhilfsmittel ausgebrannt werden und ein spongiosa-artiger Formkörper mit den kristallinen Hauptbestandteilen an Ca₂Kₓ₋₁Naₓ₊₁(PO₄)₂ (x=0,1-0,9) sowie Na₂CaP₂O₇, K₂CaP₂O₇, Ca₂P₂O₇ und/oder Mischkristalle zwischen diesen Phasen erhalten wird.

In einer bevorzugten Ausführungsform der Erfindung wird ein Teil der eingesetzten Rohstoffe separat geschmolzen, um daraus ein Glas herzustellen, das als Sinterhilfsmittel wirkt und für die spongiosa-artigen Formkörper mit besonderem Vorteil verwendet werden kann. Dieses ebenfalls aufgemahlene Glas kann dabei dem Schlicker zugesetzt werden, der aus dem erfindungsgemäß hergestellten Werkstoff besteht, der zuvor nach dem Schmelzen und Abkühlen vermahlen und dann verschlickert wurde. In Bezug auf den Feststoffgehalt im Schlicker kann das separat geschmolzene Glas in einem Anteil von 0,5-15, vorzugsweise 4-8 Gew-% zugesetzt werden, so dass jedoch die einzelnen Komponenten nicht die erfindungsgemäßen Zusammensetzungsvorgaben überschreiten. Ein derartiges Glas kann insbesondere auf der Basis von SiO₂, MgO und Na₂O gebildet werden.

Beim Sinterprozess wird bei dieser Ausführungsform eine sehr feste Struktur des Formkörpers erreicht, während beim gemeinsamen Schmelzen aller Komponenten und späteren Sintern Abbröckeln des Formkörpers auftreten kann. Das separat geschmolzene Glas hat beim Zusetzen zu dem gemahlenen Werkstoff eine Komgröße D₅₀ von 0,7 bis 7 µm, wobei der Werkstoff etwa die gleiche oder eine größere Komgröße hat.

Das oben genannte Glas als Sinterhilfsmittel für
resorbierbare calciumphosphathaltige Werkstoffe mit Ausnahme von Tricalciumphosphat hat eine chemische Zusammensetzung in Gew-% von:
SiO₂: 68 - 78, bevorzugt 73 - 78, insbesondere 74 - 75
MgO: 5 - 12, bevorzugt 8 - 11, insbesondere 8,5 - 10
Na₂O: 12 - 27, bevorzugt 12 - 19, insbesondere 14,5 - 17
K₂O: 0 - 22, vorzugsweise 0 - 5
P₂O₅: 0 - 20, vorzugsweise 0-10 hat.

Eine weitere Verarbeitungsmöglichkeit besteht darin, den Werkstoff aufzumahlen, mit üblichen Sinterhilfsmitteln zu versetzen und den so erhaltenen Schlicker zu einer Folie zu verarbeiten, die nach dem Brennprozess eine offenporige Struktur vorweist.

Vorteilhaft kann der erfindungsgemäße Werkstoff auch im Verbund mit einer metallischen Implantatoberfläche vorliegen. Da der Ausdehnungskoeffizient im Bereich von 12 bis 18x10⁻⁶ K⁻¹ liegt, gemessen mittels Dilatometer (Kieselglas-Schubstangen-Dilatometer, Fa. Netzsch, Deutschland), ist eine Anpassung an bekannte Stähle, wie z.B Chrom-Kobalt-Molybdän-Stähle mit ähnlichen Ausdehnungskoeffizienten, besonders vorteilhaft.

Die Verwendung des erfindungsgemäßen röntgenamorph-kristallinen Materials zur Herstellung von Granulaten, keramischen Formkörpern oder keramischen Folien ist ebenfalls Gegenstand der Erfindung.

Die Erfindung wird nachstehend durch Beispiele näher erläutert. Alle Prozentangaben sind auf das Gewicht bezogen, sofern nicht anderes angegeben ist.

In der dazugehörigen Zeichnung zeigen
Fig. 1: ³¹P-MAS-NMR-Spektren der erfindungsgemäßen Materialien GA1 , GA2 und GA3 mit der Zusammensetzung gemäß Beispiel 1 und den Phasen gemäß Beispiel 5 (MAS = Magic Angle Spinning);
Fig. 2: ³¹P-MAS-NMR-Spektren der erfindungsgemäßen Materialien GA4 und GA5 mit der Zusammensetzung gemäß Beispiel 2 und den Phasen gemäß Beispiel 5.

### Beispiel 1

Es wurden folgende Materialien synthetisiert nach den Vorgaben in Gewichts-%:

| Code | CaO | MgO | P₂O₅ | Na₂O | K₂O | SiO₂ |
|---|---|---|---|---|---|---|
| GA 1 | 30,67 | 2,45 | 43,14 | 9,42 | 14,32 | 0,00 |
| GA 2 | 29,92 | 2,39 | 44,53 | 9,19 | 13,97 | 0,00 |
| GA 3 | 29,21 | 2,33 | 45,85 | 8,97 | 13,64 | 0,00 |

Zum besseren Verständnis, kann man diese Schmelzabfolge auch so darstellen: GA 1; GA 2 (= GA 1 + 2,5% P₂O₅); GA 3 (= GA 1 + 5% P₂O₅). Die Gemenge wurden wie folgt eingewogen:

| Code | CaCO₃ in g | MgO in g | 85%ige-H₃PO₄ in ml | Na₂CO₃ in g | K₂CO₃ in g | SiO₂ in g |
|---|---|---|---|---|---|---|
| GA 1 | 54,74 | 2,45 | 41,48 | 16,11 | 21,01 | 0 |
| GA 2 | 53,40 | 2,39 | 42,82 | 15,72 | 20,50 | 0 |
| GA 3 | 52,13 | 2,33 | 44,09 | 15,34 | 20,01 | 0 |

Zunächst werden die Komponenten an Calcium, Magnesium, Natrium und Kalium, ggf. auch Silicium eingewogen. Nach dem Einwiegen wird das jeweilige Gemenge in einem Taumelmischer eine Stunde lang gemischt. Danach wird das Gemenge mit der 85%igen Ortho-Phosphorsäure versetzt, gut gemörsert und gerührt sowie eine Stunden lang bei 100°C getrocknet, erneut gemörsert und wiederum eine Stunde bei 100°C im Trockenschrank aufbewahrt. Sodann wurde das Gemenge erneut gemörsert und in einen Pt/Rh-Schale gefüllt und auf 400°C erhitzt, nach Erreichen dieser Temperatur eine Stunde lang bei dieser Temperatur gehalten, sodann auf 800°C hochgeheizt, nach Erreichen dieser Temperatur wiederum eine Stunde lang bei dieser Temperatur gehalten und sodann auf 1000°C erhitzt und nach Erreichen dieser Temperatur eine Stunde lang bei dieser Temperatur gehalten. Dieser Sinterkuchen wurde an Luft abgekühlt und erneut zum Zwecke der Homogenisierung gemörsert. Dieses vorbehandelte Gemenge wurde sodann in einen Platin-Tiegel gefüllt und im Schmelzofen auf 1600°C erhitzt. Nach Erreichen dieser Temperatur wurde die Schmelze eine halbe Stunde lang bei dieser Temperatur belassen. Die dünnflüssigen homogenen Schmelzen wurden dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe.

### Beispiel 2

Nach dem gleichen Herstellungsprozedere, wie in Beispiel 1 beschrieben, d.h. Gemengeerzeugung mit Calciumcarbonat, Natriumcarbonat, Kaliumcarbonat und Ortho-Phosphorsäure wurden folgende Zusammensetzungen nach den folgenden Vorgaben in Gewichts-%:

| Code | CaO | MgO | P₂O₅ | Na₂O | K₂O | SiO₂ |
|---|---|---|---|---|---|---|
| GA 4 | 31,54 | 1,19 | 42,37 | 9,17 | 13,95 | 1,78 |
| GA 5 | 30,79 | 1,16 | 43,74 | 8,95 | 13,62 | 1,73 |

Es ergaben sich für alle Zusammensetzungskompositionen dünnflüssige Schmehen, die spontan beim Abkühlen kristallisierten. Die Kristallisationsprodukte wiesen eine weiße Farbe auf.

### Beispiel 3

Eine weitere Herstellungsmöglichkeit besteht u.a. darin, dass die gesamte Phosphor- bzw. Phosphatmenge oder wie im vorliegenden Beispiel ein Teil durch einen Calciumträger eingebracht werden kann. Es wurde folgende Zusammensetzung synthetisiert nach den Vorgaben in Gewichts-%:

| Code | CaO | MgO | P₂O₅ | Na₂O | K₂O | SiO₂ |
|---|---|---|---|---|---|---|
| GA 1 | 30,67 | 2.45 | 43,14 | 9,42 | 14,32 | 0,00 |

Das Gemenge wurde wie folgt eingewogen:

| Code | CaCO₃ in g | Magnesium-hydroxidcarbonat in g | 85%ige-H₃PO₄ in ml | Na₂CO₃ in g | K₂CO₃ in g | CaHPO₄ in g |
|---|---|---|---|---|---|---|
| GA 1 | 0,00 | 5,13 | 4,25 | 16,11 | 21,00 | 74,43 |

Das Gemenge wurde nach dieser Vorgabe eingewogen, eine Stunde lang im Taumelmischer gemischt, mit der Phosphorsäure versetzt, eine Stunde bei 100°C getrocknet, an der Luft abgekühlt und gemörsert. Dieses Gemenge wurde in einen Platin-Tiegel gefüllt und in einen auf 450°C vorgewärmten Ofen gefüllt und 6 Std. gehalten, dann in einen auf 800°C vorgeheizten Ofen gestellt und 16 Stunden lang bei dieser Temperatur gehalten. Der Tiegel wurde entnommen und der Ofen nunmehr auf 950°C vorgeheizt. In den auf 950°C vorgeheizten Ofen wurde der Tiegel 6 Stunden lang gehalten. Sodann wurde die Probe auf 1600°C hochgeheizt und eine halbe Stunde nach Erreichen dieser Temperatur bei dieser Temperatur gehalten. Die dünnflüssige homogene Schmelze wurde dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe. In Abhängigkeit von der eingesetzten CaHPO₄-Komponente und deren Verunreinigung an Eisen und/oder Mangan, kann eine Verfärbung beobachtet werden.

Es ist auch möglich die Schmelze nach dem Schmelzvorgang (1600°C, 0,5h).direkt in einem Wasserbad abzuschrecken (Fritten), um im Falle einer weiteren Verarbeitung im Schlickerform das weitere Zerkleinern des Schmelzproduktes zu erleichtern.

### Beispiel 4

Zur Bestimmung der Löslichkeit wurde von den Proben nach Beispiel 1 und ausgewählten Proben nach Beispiel 2 (siehe folgende Tabelle) Granulate in der Komfraktion 315µm bis 400µm hergestellt. Als Lösungsmittel wurde eine 0,2M TRIS-HCI-Puffer-Lösung mit pH=7,4 bei 37°C verwendet. Die geprüfte Menge betrug 50mg auf 40ml Lösungsmittel. Die Lagerung der Granulate erfolgte bei 37°C über eine Zeitdauer von 120h. Danach wurde die Gesamtlöslichkeit durch Bestimmung der Einzelionen (von Ca, Mg, P, Na, K) in der Lösung mit Hilfe einer ICP-Messung bestimmt zu:

| Code | Löslichkeit [µg/mg] |
|---|---|
| GA 1 | 95±8 |
| GA 2 | 134±16 |
| GA 3 | 221±22 |
| GA 4 | 90±8 |
| GA 5 | 152±10 |

### Beispiel 5

Von den Proben nach Beispiel 1 und nach Beispiel 2 wurden ³¹P-MAS-NMR-Spektren mit einer Wartezeit von 120s zwischen den Einzelpulsen aufgenommen.
Die Probenrotationsgeschwindigkeit betrug 12,5 kHz.
Die quantitative Zusammensetzung der Proben in Bezug auf den Phosphatanteil ist folgender Tabelle angegeben:

| Code | Orthophosphat-Anteil [(PO₄)³] in % | Diphosphat-Anteil [(P₂O₇)²⁻] in % | Kettenphosphat-Anteil [vorrangig (PO₃)¹] in % |
|---|---|---|---|
| GA 1 | 99,5-96 | 0,5-4 | - |
| GA 2 | 88 | 12 | - |
| GA 3 | 79 | 21 | - |
| GA 4 | 95 | 5 | - |
| GA 5 | 89 | 11 | - |

Während von den angegebenen Proben jeweils nur eine Probe untersucht wurde, bezieht sich das Ergebnis mit der angegebenen Bandbreite für die Zusammensetzung GA 1 auf insgesamt drei Chargen, wobei eine Charge nach der unter Beispiel 3 genannten Herstellungsmethode synthetisiert wurde.

### Beispiel 6

In einer Planetenmühle im Zirkonoxidbecher (250ml) wurde unter den Bedingungen zwei Mal 20min lang das geschmolzenen Produkte der mit der Code-Bezeichnung Zusammensetzungen GA 1 aufgemahlen. Das Resultate ist in der folgenden Tabelle zu dargestellt.

| Code | D₅₀-Wert [in µm] |
|---|---|
| GA 1 | 6,50 |

### Beispiel 7

Die aufgemahlene Probe GA 1 nach Beispiel 6 soll zu "scaffolds" verarbeitet werden. Von diesem gemahlenen Gut wurden 100g mit 45g eines Gemisches, bestehend aus 90% Polyethylenglykol und 10% eines handelsüblichen Netzmittels, unter Zugabe von 5ml Isopropylalkohol zu einem Schlicker verarbeitet Dieser Schlicker wird auf PUR-Schwämme (PUR=Polyurethan) mit offener Porosität mit 31,49-7,87 Poren/cm (80 bis 20 ppi - Poren pro Zoll) durch mehrmaliges Eintauchen und Ausdrücken aufgebracht, im Trockenschrank über Nacht bei 120°C getrocknet und dann langsam mit 10°C pro Minute auf 1000°C erhitzt. Im Ergebnis ist ein spongiosa-artiges Material mit dem Ausgangsschwamm ähnlichem Aufbau vorhanden, und der PUR-Schwamm ist rückstandslos ausgebrannt.

### Beispiel 8

Um die Festigkeit der spongiosa-arügen Formkörper noch weiter zu erhöhen, wurde als Sinterhilfsmittel 3-Masse-% eines zuvor hergestellten Glases mit der chemischen Zusammensetzung in Masse-% SiO₂: 74,97; MgO: 9,22 und Na₂O: 15,81 (eingeschmolzen als 27,04 Na₂CO₃) und einem D₅₀ von 6,56µm dem Mahlgut von GA 1 zugegeben. Von diesem Pulvergemisch wurden 100g mit 45g eines Gemisches, bestehend aus 90% Polyethylenglykol und 10% eines handelsüblichen Netzmittels, unter Zugabe von 5ml Isopropylalkohol zu einem Schlicker versetzt. Dieser Schlicker wird auf PUR-Schwämme mit offener Porosität mit 31,49-7,87 Poren/cm (80 bis 20 ppi Poren pro Inch) durch mehrmaliges Eintauchen und Ausdrücken aufgebracht, im Trockenschrank über Nacht bei 120°C getrocknet und dann langsam mit 10°C pro Minute auf 1000°C erhitzt. Im Ergebnis ist ein spongiosa-artiges Material mit dem Ausgangsschwamm ähnlichem Aufbau vorhanden, und der PUR-Schwamm ist rückstandslos ausgebrannt.

### Beispiel 9

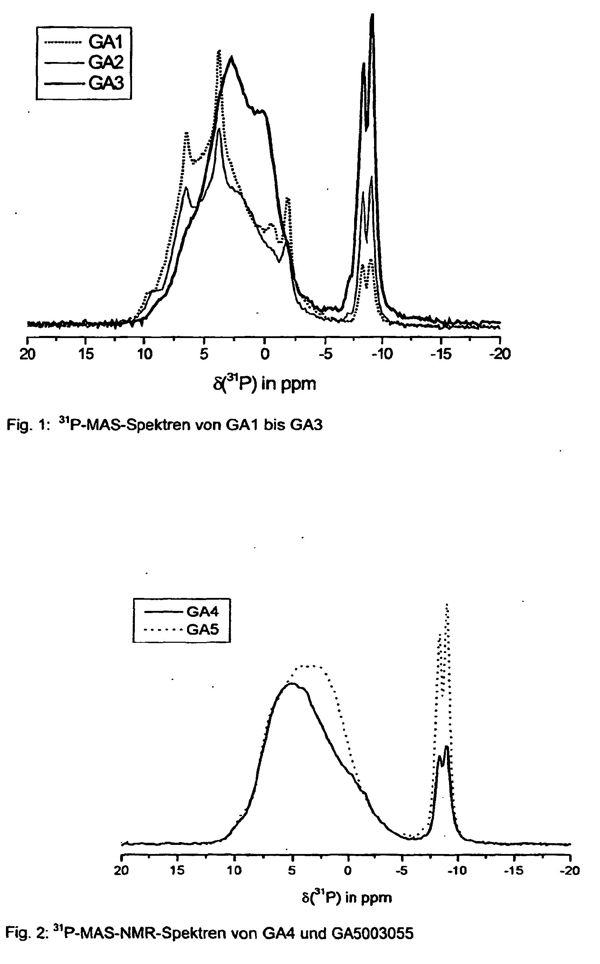

Es wurden Proben nach Beispiel 1 und Beispiel 2 hergestellt und mit Hilfe der ³¹P-NMR untersucht. Die ³¹P-MAS-NMR-Spektren wurden mit einer Wartezeit von 120s zwischen den Einzelpulsen aufgenommen. Die Probenrotationsgeschwindigkeit betrug 12,5 kHz.
Im Ergebnis kann gezeigt werden, dass die Proben GA 1 bis GA 3 (vergl. Fig. 1), die sich chemisch nur durch die Erhöhung des Phosphat-Anteils unterscheiden, analog die Proben GA4 und GA 5 (vergl. Fig. 2), diesen erhöhten Phosphatanteil in einem röntgenamorph-kristallinen Diphosphatanteil im Schmelzprodukt wiederspiegeln, was auch die Löslichkeit dramatisch beeinflusste (vergl. Beispiel 4).

Die Spektren von Fig. 1 und 2 zeigen jeweils in dem linken (breiten) Peak die Q₀-Gruppen an und in dem rechten (schmaleren) Peak die Q₁-Gruppen.

### Beispiel 10

Material der Zusammensetzung mit der Code-Bezeichnung GA 1 wurde frisch aufgemahlen und 1g einer Komfraktion von <45µm wurden in 100ml E-pur-Wasser gegeben und nach 1 min und nach 72h der pH-Wert bestimmt. Das Ergebnis nach einer Minute war 10,55 und nach 72 Stunden 8,71, d.h. deutlich in Richtung der physiologischen Bedingungen verschoben.

### Beispiel 11

Um diesen Effekt a priori zu verstärken, wurde folgender Versuch durchgeführt. Es wurde ein spongiosa-artiger Formkörper hergestellt nach Beispiel 7, d.h. also die Zusammensetzung der Code-Bezeichnung GA 1, und in dem vorliegenden Beispiel gesintert auf einen PUR-Ausgangsschwamm von 30ppi.
Dieser Formkörper von den äußeren Abmaßen ca. 11mmx11mmx7mm wurde in 100ml E-pur-Wasser gegeben und der pH-Wert nach 10min gemessen. Diese Messung ergab einen Wert von 9,62.
Sodann wurde der Formkörper bei 60°C und einem Druck von 3 bar in E-pur-Wasser eine Stunde lang eluiert. Nach 5-fachen Spülen in 20ml frischem E-pur-Wasser wurde der Formkörper erneut in 100ml E-pur-Wasser gegeben und der pH-Wert nach 1 Stunden zu 8,83 gemessen.
Damit wird deutlich, dass eine derartige Vorbehandlung von spongiosa-artigen Formkörpern eine sinnvolle Verfahrensweise darstellt, da danach vorbehandelte Produkte eine geringere Basizität vorweisen, was sich sowohl bei der Implantation unter Bedingungen in vivo als auch beim Tissue Engineering unter Bedingungen ex vivo respektive in vitro als vorteilhaft erweisen kann.

### Beispiel 12

Im Hinblick auf eine Beschichtung von Werkstoffen mit diesen resorbierbaren erfindungsgemäßen Werkstoffen ist es von Bedeutung, dass der thermische Ausdehnungskoeffizient variiert werden kann, wenn man zum Beispiel bedenkt, dass Titanimplantate um 8•10⁻⁶ K⁻¹ und Co-Cr-Mo-Stähle um 14-16•10⁻⁶·K⁻¹ (abhängig von den Legierungsbestandteilen) aufweisen. Um ein für den jeweiligen Anwendungszweck optimalen Verbund zu erzeugen, spielt es eine Rolle, in welchem Temperaturbereich der Werkstoff auf das metallische Substrat aufgebracht wird, da man auf diese Weise auch gezielt das Substrat durch die Beschichtung unter Druckspannungen, d.h. unter Vorerhitzen setzen kann, was im Allgemeinen als mechanisch stabilerer Verbund angesehen wird.

Die folgende Tabelle zeigt nun einen Ausschnitt aus der Variationsmöglichkeit:

| Probe | **AK**_{**30-100**} (10⁻⁶ K⁻¹) | **AK**_{**RT-400**} (10⁻⁶K⁻¹) | **AK** _{**50-400**} (10⁻⁶ K⁻¹) |
|---|---|---|---|
| GA 1 | 12,15 | 14,84 | 15,14 |
| GA 2 | 13,64 | 17,16 | 17,54 |
| GA 3 | 13,21 | 16,99 | 17,45 |
| GA 4 | 12,51 | 15,85 | 16,20 |
| GA 5 | 13,29 | 16,69 | 17,08 |

AK₃₀₋₁₀₀ bedeutet dabei der Ausdehnungskoeffizient zwischen 30 und 100 °C,
AK_{RT**400} ist der Ausdehnungskoeffizient zwischen Raumtemperatur (25) und 400 °C, und AK₅₀₋₄₀₀ ist der Ausdehnungskoeffizient zwischen 50 und 400 °C .

## Patentansprüche

1. Knochenersatzwerkstoff mit kristallinen und röntgenamorphen Phasen, **dadurch gekennzeichnet, dass**
a) der Knochenersatzwerkstoff nach ³¹P-NMR-Messungen Q₀-Gruppen von Orthophosphat und Q₁-Gruppen von Diphosphat umfaßt, wobei die Orthophosphate respektive Q₀-Gruppen, bezogen auf den Gesamtphosphorgehalt des fertigen Werkstoffes, 70 bis 99,9 Gew-% betragen, und die Diphosphate respektive Q₁-Gruppen, bezogen auf den Gesamtphosphorgehalt des fertigen Werkstoffes, 0,1 bis 30 Gew-% betragen, und
b) nach röntgendiffraktometrischen Messungen, bezogen auf das Gesamtgewicht des fertigen Werkstoffes, 30 bis 99,9 Gew-% einer Hauptkristallphase aus Ca₂K₁₋ₓNa₁₊ₓ(PO₄)₂ mit x=0,1 bis 0,9 in dem Knochenersatzwerkstoff enthalten sind, und als Nebenkristallphase, bezogen auf das Gesamtgewicht des fertigen Werkstoffes, 0,1 bis 20 Gew-% eines Stoffes enthalten sind, ausgewählt aus der Gruppe, bestehend aus Na₂CaP₂O₇, K₂CaP₂O₇, Ca₂P₂O₇ und Gemische davon, und
c) die röntgenamorphen Phasen neben der Hauptkristallphase insgesamt 0,1 bis 70 Gew-% betragen, bezogen auf das Gesamtgewicht des fertigen Werkstoffes, erhältlich durch
Vermischen von Rohstoffen, enthaltend (in Gew-%) 25-50 CaO, 1-20 Na₂O, 0,5-20 K₂O, 0-13 MgO, 0-10 SiO₂ und Behandlung mit H₃PO₄ entsprechend einem Anteil von 30-55 P₂O₅, wobei SiO₂ oder MgO oder ein Gemisch davon wenigstens 1 Gew-% beträgt, das Gemenge homogenisiert und getrocknet wird und einer stufenweisen Temperaturbehandlung von jeweils 1-2h bei 350-450 °C, 750-850 °C und 950-1050 °C unterworfen wird, und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

2. Knochenersatzwerkstoff nach Anspruch 1, **dadurch gekennzeichnet, dass** zusätzlich 0,1-10 Gew-% ein oder mehrere Kettenphosphate NaPO₃, KPO₃ und Gemische davon enthalten sind, wobei diese nach ³¹P-NMR-Messungen als Q₂-Gruppen nachzuweisen sind.

3. Knochenersatzwerkstoff nach Anspruch 1, **dadurch gekennzeichnet, dass** x im Bereich von 0,2 bis 0,9 liegt.

4. Knochenersatzwerkstoff nach Anspruch 1, **dadurch gekennzeichnet, dass** in der Nebenkristallphase entsprechend dem SiO₂-Anteil eine Silicatphase enthalten ist.

5. Knochenersatzwerkstoff nach Anspruch 1, **dadurch gekennzeichnet, dass** zusätzlich Magnesium bis zu einem Anteil von 10 Gew-%, berechnet als MgO und bezogen auf das Gewicht des fertigen Werkstoffs, enthalten ist

6. Knochenersatzwerkstoff nach Anspruch 1, **dadurch gekennzeichnet, dass** der Anteil der Orthophosphatphase als Q₀-Gruppen im Bereich von 75 bis 99 Gew-% liegt, vorzugsweise im Bereich von 78 bis 95 Gew-%.

7. Knochenersatzwerkstoff nach Anspruch 1, **dadurch gekennzeichnet, dass** der Anteil der Diphosphatphase als Q₁-Gruppen im Bereich von 1 bis 22 Gew-% liegt, vorzugsweise im Bereich von 5 bis 16 Gew-%.

8. Knochenersatzwerkstoff nach Anspruch 1, **dadurch gekennzeichnet, dass** der Anteil der Nebenkristailphase 0,1 bis 15 Gew-% beträgt, vorzugsweise 1 bis 15 Gew-%.

9. Knochenersatzwerkstoff nach einem der Ansprüche 1 bis 8, **dadurch gekennzeichnet, dass** die Gesamtlöslichkeit, bezogen auf den Ausgangswerkstoff, im Bereich von 30 bis 500 µg/mg, vorzugsweise 150-400 µg/mg liegt, gemessen in 0,2M TRIS-HCl-Puffer-Lösung bei pH=7,4, T=37°C, an einer Komfraktion von 315 - 400µm, 120h lang bei einem Verhältnis von 50mg Probeneinwaage zu 40ml Pufferlösung.

10. Knochenersatzwerkstoff nach einem der Ansprüche 1 bis 8, **dadurch gekennzeichnet, dass** der Ausdehnungskoeffizient im Bereich von 12 bis 18x10⁻⁶ K⁻¹ liegt, gemessen mittels Dilatometer.

11. Knochenersatzwerkstoff nach einem der Ansprüche 1 bis 8, **dadurch gekennzeichnet, dass** die Oberfläche nach Lagerung in deionisiertem Wasser bei Raumtemperatur über 72 Stunden oder Erhitzen auf 60 °C über 1 Stunde bei 1-3 bar und Spülen mit deionisiertem Wasser eine pH-Änderung im alkalischen Bereich um wenigstens 0,7 Einheiten, vorzugsweise wenigstens 1,5 Einheiten in Richtung zum Neutralpunkt zeigt.

12. Knochenersatzwerkstoff nach Anspruch 1, **dadurch gekennzeichnet, dass** der Werkstoff im Verbund mit einer metallischen Implantatoberfläche vorliegt.

13. Knochenersatzwerkstoff nach Anspruch 1, **dadurch gekennzeichnet, dass** er im verarbeiteten, fertigen Zustand besteht aus (in Gew-%):
30 bis 55 P₂O₅, 25 bis 50 CaO, 1 bis 20 Na₂O, 0,5 bis 20 K₂O, 0 bis 13 MgO bevorzugt 1-13 MgO, 0 bis 10 SiO₂ bevorzugt 0,5-5 SiO₂ , wobei MgO oder SiO₂ oder ein Gemisch davon wenigstens 1 Gew-% beträgt.

14. Knochenersatzwerkstoff nach Anspruch 13, **dadurch gekennzeichnet, dass** er enthält 40 bis 52 P₂O₅, 28 bis 33 CaO, 8,5 bis 13 Na₂O, 9,5 bis 15 K₂O, 1,5 bis 3 MgO, 0,1 bis 4 SiO₂.

15. Knochenersatzwerkstoff nach Anspruch 1, **dadurch gekennzeichnet, dass** er in Form von Granulaten, keramischen Formkörpern oder keramischen Folien vorliegt

16. Verfahren zur Herstellung eines Knochenersatzwericstoffes mit kristallinen und röntgenamorphen Phasen nach Anspruch 1, **dadurch gekennzeichnet, dass** Rohstoffe, enthaltend (in Gew-%) 25-50 CaO, 1-20 Na₂O, 0,5-20 K₂O, 0-13- MgO, 0-10 SiO₂ vermischt und mit H₃PO₄ entsprechend einem Anteil von 30-55 P₂O₅ behandelt werden, wobei MgO oder SiO₂ oder ein Gemisch davon wenigstens 1 Gew-% beträgt, das Gemenge homogenisiert und getrocknet wird und einer stufenweisen Temperaturbehandlung von jeweils 1-2h bei 350-450 °C, 750-850 °C und 950-1050 °C unterworfen wird, und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

17. Verfahren nach Anspruch 16, **dadurch gekennzeichnet, dass** die Rohstoffe in zwei Chargen aufgeteilt und separat geschmolzen werden, wobei die erste Charge aus einem geschmolzenen Glas aus 73-78 SiO₂, 8-11 MgO, 8,5-10 Na₂O, 12-19 K₂O und 0-22 P₂O₅ (in Gew-%) besteht, das nach dem Abkühlen aufgemahlen und in einem Anteil von 0,1-15 Gew-% der zweiten Charge, die das röntgenamorph-kristalline Material von Anspruch 16 darstellt, zugegeben wird und mit diesem zu Formkörpern bei 900-1200 °C gesintert wird.

18. Verfahren nach Anspruch 16, **dadurch gekennzeichnet, dass** das Gemisch bei 1590 bis 1650 °C geschmolzen wird.

## Claims

1. A bone replacement material comprising crystalline and X-ray amorphous phases, **characterized in that**
a) according to ³¹P-NMR measurements, the bone replacement material comprises Q₀-groups of orthophosphate and Q₁-groups of diphosphate, the orthophosphates or Q₀-groups making up 70 to 99.9% by weight relative to the total phosphorus content of the finished material and the diphosphates or Q₁-groups making up 0.1 to 30% by weight relative to the total phosphorus content of the finished material, and
b) according to X-ray diffractometric measurements and relative to the total weight of the finished material, 30 to 99.9% by weight of a main crystal phase consisting of Ca₂K₁₋ₓNa₁₊ₓ(PO₄)₂, where x = 0.1 to 0.9, is contained in the bone replacement material and 0.1 to 20% by weight of a substance selected from the group consisting of Na₂CaP₂O₇, K₂CaP₂O₇, Ca₂P₂O₇ and mixtures thereof is contained as a secondary crystal phase, and
c) the X-ray amorphous phases contained besides the main crystal phase jointly make up 0.1 to 70% by weight relative to the total weight of the finished material, obtainable by
mixing raw materials containing (in % by weight) 25-50 CaO, 1-20 Na₂O, 0.5-20 K₂O, 0-13 MgO and 0-10 SiO₂ and treating the aforesaid mixture with H₃PO₄ in an amount corresponding to 30-55 P₂O₅, SiO₂ or MgO or a mixture thereof making up at least 1 % by weight, homogenizing and drying the mixture and subjecting it to a step-by-step thermal treatment lasting 1-2h at 350-450°C, 750-850°C and 950-1,050°C respectively, melting the mixture at between 1,550 and 1,650°C, holding it at the melting temperature for between 10 and 60 minutes and finally cooling the mixture in a spontaneous or temperature-controlled manner, grinding it, if necessary, and sintering it to obtain moulded bodies.

2. A bone replacement material according to Claim 1, wherein additionally one or more of the chain phosphates NaPO₃, KPO₃ and mixtures thereof are contained in an amount ranging between 0.1 and 10% by weight, which chain phosphates can be detected as Q₂-groups using ³¹P-NMR measurements.

3. A bone replacement material according to Claim 1, wherein x ranges between 0.2 and 0.9.

4. A bone replacement material according to Claim 1, wherein the secondary crystal phase contains a silicate phase corresponding to the SiO₂ content.

5. A bone replacement material according to Claim 1, wherein additionally magnesium in an amount ranging up to 10% by weight, calculated as MgO and relative to the weight of the finished material, is contained.

6. A bone replacement material according to Claim 1, wherein the orthophosphate phase represented by Q₀-groups is in the range of 75 to 99% by weight, preferably in the range of 78 to 95% by weight.

7. A bone replacement material according to Claim 1, wherein the diphosphate phase represented by Q₁-groups is in the range of 1 to 22% by weight, preferably in the range of 5 to 16% by weight.

8. A bone replacement material according to Claim 1, wherein the secondary crystal phase is in the range of 0.1 to 15% by weight, preferably in the range of 1 to 15% by weight.

9. A bone replacement material according to any of Claims 1 through 8, wherein the total solubility ranges between 30 and 500µg/mg, relative to the starting material if the test is carried out in 0.2M TRIS-HCl buffer solution at pH = 7.4, T = 37°C using a grain size fraction of 315-400µm, the duration of the test being 120h and the ratio of weighed-in sample to buffer solution being 50mg to 40ml.

10. A bone replacement material according to any of Claims 1 through 8, wherein the coefficient of expansion ranges between 12 and 18 x 10⁻⁶ K⁻¹, measured using a dilatometer.

11. A bone replacement material according to any of Claims 1 through 8, wherein the pH value of the surface changes by at least 0.7 units, preferably at least 1.5 units, towards the neutral point within the alkaline range if the material is stored in deionized water at room temperature for 72 hours or heated up to 60°C for 1 hour at a pressure of 1-3 bars and rinsed with deionized water.

12. A bone replacement material according to Claim 1, **characterized in that** said material is provided in combination with a metallic implant surface.

13. A bone replacement material according to Claim 1, wherein in the processed, finished state said material consists of (in % by weight):
30 to 55 P₂O₅, 25 to 50 CaO, 1 to 20 Na₂O, 0.5 to 20 K₂O, 0 to 13 MgO, preferably 1 to 13 MgO, 0 to10 SiO₂, preferably 0.5 to 5 SiO₂; MgO or SiO₂ or a mixture thereof making up at least 1% by weight.

14. A bone replacement material according to Claim 13, wherein said material contains 40 to 52 P₂O₅, 28 to 33 CaO, 8.5 to 13 Na₂O, 9.5 to 15 K₂O, 1.5 to 3 MgO, 0.1 to 4 SiO₂.

15. A bone replacement material according to Claim 1, wherein said material is provided in the form of granulated materials, ceramic bodies or ceramic sheets.

16. A method for manufacturing a bone replacement material comprising crystalline and X-ray amorphous phases according to Claim 1, **characterized by** mixing raw materials containing (in % by weight) 25-50 CaO, 1-20 Na₂O, 0.5-20 K₂O, 0-13 MgO and 0-10 SiO₂ and treating the aforesaid mixture with H₃PO₄ in an amount corresponding to 30-55 P₂O₅, MgO or SiO₂ or a mixture thereof making up at least 1% by weight, homogenizing and drying the mixture and subjecting it to a step-by-step thermal treatment lasting 1-2h at 350-450°C, 750-850°C and 950-1,050°C respectively, melting the mixture at between 1,550 and 1,650°C, holding it at the melting temperature for between 10 and 60 minutes and finally cooling the mixture in a spontaneous or temperature-controlled manner, grinding it, if necessary, and sintering it to obtain moulded bodies.

17. A method according to Claim 16, wherein the raw materials are divided into two batches and melted separately, the first batch consisting of a melted glass comprising 73-78 SiO₂, 8-11 MgO, 8.5-10 Na₂O, 12-19 K₂O and 0-22 P₂O₅ (in % by weight), which glass is cooled, ground and added to the second batch, i.e. the X-ray amorphous-crystalline material of claim 16, in an amount ranging between 0.1 and 15% by weight and is sintered jointly with said second batch at 900-1,200°C to obtain moulded bodies.

18. A method according to Claim 21, wherein the mixture is melted at between 1,590 and 1,650°C.

## Revendications

1. Matériau pour prothèse osseuse avec des phases cristallines et amorphes, **caractérisé en ce que**
a) le matériau pour prothèse osseuse comprend, par des mesures de RMN ³¹P, des groupes Q₀ d'orthophosphate et des groupes Q₁ de diphosphate, où les groupes Q₀ d'orthophosphate s'élèvent de 70 à 99,9 % en poids, sur base de la teneur totale en phosphore du matériau fini, et les groupes Q₁ de diphosphate s'élèvent de 0,1 à 30 % en poids, sur base de la teneur totale en phosphore du matériau fini et
b) par des mesures de diffraction des rayons X, sur base du poids total du matériau fini, 30 à 99,9 % en poids d'une phase cristalline principale en Ca₂K₁₋ₓNa₁₊ₓ(PO₄)₂ avec x = 0,1 à 0,9 sont présents dans le matériau pour prothèse osseuse, et comme phase cristalline secondaire, sur base du poids total du matériau fini, 0,1 à 20 % en poids d'une substance sont présents, laquelle substance est choisie parmi le groupe consistant en Na₂CaP₂O₇, K₂CaP₂O₇, Ca₂P₂O₇, et leurs mélanges, et
c) les phases amorphes, en plus de la phase cristalline principale, s'élèvent au total, de 0,1 à 70 % en poids, sur base du poids total du matériau fini,
pouvant être obtenu par mélange des matières premières, contenant (en % en poids), 25-50 CaO, 1-20 Na₂O, 0,5-20 K₂O, 0-13MgO, 0-10 SiO₂ et traitement avec H₃PO₄, correspondant à une quantité de 30-55 P₂O₅, où SiO₂ ou MgO ou un mélange de ceux-ci composé au moins 1 % en poids, le mélange est homogénéisé et séché, puis soumis à un traitement thermique graduel de chaque fois 1-2 heures, à 350-450°C, 750-850°C et 950-1050°C, et le mélange est fondu de 1550 à 1650°C, maintenu à la température de fusion pendant 10 à 60 minutes et finalement, refroidi spontanément ou avec une température réglée et le cas échéant, broyé et fritté en un corps moulé.

2. Matériau pour prothèse osseuse selon la revendication 1, **caractérisé en ce qu'**il contient en outre, 0,1-10 % en poids d'un ou de plusieurs phosphates linéaires NaPO₃, KPO₃ et leurs mélanges, où ceux-ci sont détectés par mesures de RMN ³¹P comme des groupes Q₂.

3. Matériau pour prothèse osseuse selon la revendication 1, **caractérisé en ce que** x se situe dans l'intervalle allant de 0,2 à 0,9.

4. Matériau pour prothèse osseuse selon la revendication 1, **caractérisé en ce que** dans la phase cristalline secondaire, une phase silicate est présente, correspondant à la quantité de SiO₂.

5. Matériau pour prothèse osseuse selon la revendication 1, **caractérisé en ce qu'**en outre, il contient du magnésium en une quantité de jusqu'à 10 % en poids, calculé comme MgO et par rapport au poids du matériau fini.

6. Matériau pour prothèse osseuse selon la revendication 1, **caractérisé en ce que** la quantité de phase orthophosphate, comme groupes Q₀, se situe dans l'intervalle allant de 75 à 99 % en poids, de préférence dans l'intervalle allant de 78 à 95 % en poids.

7. Matériau pour prothèse osseuse selon la revendication 1, **caractérisé en ce que** la quantité de phase diphosphate, comme groupes Q₁, se situe dans l'intervalle allant de 1 à 22 % en poids, de préférence dans l'intervalle allant de 5 à 16 % en poids.

8. Matériau pour prothèse osseuse selon la revendication 1, **caractérisé en ce que** la quantité de phase cristalline secondaire se situe dans l'intervalle allant de 0,1 à 15 % en poids, de préférence dans l'intervalle allant de 1 à 15 % en poids.

9. Matériau pour prothèse osseuse selon l'une quelconque des revendications 1 à 8, **caractérisé en ce que** la solubilité totale, sur base du matériau de départ, se situe dans l'intervalle allant de 30 à 500 µg/mg, de préférence de 150-400 µg/mg, mesurée dans une solution tampon Tris-HCl 0,2 M à pH = 7,4, T = 37°C, pour une fraction des grains de 315-400 µm, pendant 120 heures à un rapport de 50 mg d'échantillon pesé pour 40 ml de solution tampon.

10. Matériau pour prothèse osseuse selon l'une quelconque des revendications 1 à 8, **caractérisé en ce que** le coefficient d'allongement se situe dans l'intervalle allant de 12 à 18 x 10⁶ K⁻¹, mesuré par un dilatomètre.

11. Matériau pour prothèse osseuse selon l'une quelconque des revendications 1 à 8, **caractérisé en ce que** la surface, après stockage dans de l'eau déminéralisée à la température ambiante pendant 72 heures ou avec chauffage à 60°C pendant 1 heure sous 1-3 bar et rinçage avec de l'eau déminéralisée, présente une modification du pH dans la zone alcaline d'au moins 0,7 unité, de préférence au moins 1,5 unité en direction du point de neutralisation.

12. Matériau pour prothèse osseuse selon la revendication 1, **caractérisé en ce que** le matériau est présent en liaison avec une surface métallique d'implant.

13. Matériau pour prothèse osseuse selon la revendication 1, **caractérisé en ce qu'**il consiste, à l'état traité, fini, en (% en poids) : 30 à 55 P₂O₅, 25 à 50 CaO, 1 à 20 Na₂O, 0,5 à 20 K₂O, 0 à 13 MgO, de préférence 1-13 MgO, 0 à 10 SiO₂, de préférence 0,5-5 SiO₂, où MgO ou SiO₂ ou un mélange de ceux-ci s'élève à au moins 1 % en poids.

14. Matériau pour prothèse osseuse selon la revendication 13, **caractérisé en ce qu'**il contient 40 à 52 P₂O₅, 28 à 33 CaO, 8,5 à 13 Na₂O, 9,5 à 15 K₂O, 1,5 à 3 MgO, 0,1 à 4 SiO₂.

15. Matériau pour prothèse osseuse selon la revendication 1, **caractérisé en ce qu'**il se présente sous la forme de granulés, articles moulés céramiques ou feuilles céramiques.

16. Procédé de préparation d'un matériau pour prothèse osseuse avec phases cristallines et amorphes selon la revendication 1, **caractérisé en ce que** l'on mélange les matières premières, contenant (en % en poids), 25-50 CaO, 1-20 Na₂O, 0,5-20 K₂O, 0-13 MgO, 0-10 SiO₂ et on traite avec H₃PO₄, correspondant à une quantité de 30-55 P₂O₅, où SiO₂ ou MgO ou un mélange de ceux-ci compose au moins 1 % en poids, le mélange est homogénéisé et séché, puis soumis à un traitement thermique graduel de chaque fois 1-2 heures, à 350-450°C, 750-850°C et 950-1050°C, et le mélange est fondu de 1550 à 1650°C, maintenu à la température de fusion pendant 10 à 60 minutes et finalement, refroidi spontanément ou avec une température réglée et le cas échéant, broyé et fritté en un corps moulé.

17. Procédé selon la revendication 16, **caractérisé en ce que** les matières premières sont divisées en deux charges et fondues séparément, où la première charge consiste en un verre fondu de 73-78 SiO₂, 8-11 MgO, 8,5-11 Na₂O 12-19 K₂O et 0-22 P₂O₅ (en % en poids), qui est broyée après le refroidissement, et est ajoutée à une quantité de 0,1-15 % en poids de la deuxième charge, qui représente le matériau amorphe-cristallin de la revendication 16, et frittée avec celle-ci, en un corps moulé à 900-1200°C.

18. Procédé selon la revendication 16, **caractérisé en ce que** le mélange est fondu de 1590 à 1650°C.
